# EUROPÄISCHE PATENTSCHRIFT

(11) **EP 2 853 869 B1**
(45) Veröffentlichungstag und Bekanntmachung des Hinweises auf die Patenterteilung: **31.10.2018**
(21) Anmeldenummer: 13185868.0
(22) Anmeldetag: 25.09.2013
(51) Int. Cl.: G01J 3/433, G01N 21/39

(54) **Verfahren und Gasanalysator zur Messung der Konzentration einer Gaskomponente in einem Messgas**
Method and gas analyser for measuring the concentration of a gas component in a gas to be measured
Procédé et analyseur de gaz pour la mesure de la concentration d'un composant de gaz dans un gaz de mesure

(43) Veröffentlichungstag der Anmeldung: 01.04.2015
(73) Patentinhaber: Siemens Aktiengesellschaft, 80333 München (DE)
(72) Erfinder: Steinbacher, Franz, 76137 Karlsruhe (DE)

(56) Entgegenhaltungen:
- EP-B1- 1 475 618
- DE-B3-102012 202 893
- CASSIDY D T ET AL: "HARMONIC DETECTION WITH TUNABLE DIODE LASERS - TWO-TONE MODULATION", APPLIED PHYSICS B, Bd. B29, Nr. 4, 1. Dezember 1982 (1982-12-01), Seiten 279-285, XP008047327,

## Beschreibung

Die Erfindung betrifft ein Verfahren gemäß dem Oberbegriff des Anspruchs 1 sowie einen Gasanalysator gemäß dem Oberbegriff des Anspruchs 6.

Ein derartiges Verfahren bzw. ein derartiger Gasanalysator in Form eines Laserspektrometers sind aus Cassidy D. T. et al: "Harmonic Detection with Tunable Diode Lasers - Two-Tone Modulation", Applied Physics B, Bd. 29, Nr. 4, 1. Dezember 1982, Seiten 279-285 bekannt.

Laserspektrometer, wie z. B. aus der EP 1 475 618 B1 bekannt, werden insbesondere für die optische Gasanalyse in der Prozessmesstechnik eingesetzt. Eine wellenlängenabstimmbaren Lichtquelle in Form einer Laserdiode erzeugt Licht im Infrarotbereich, das durch ein zu messendes Prozessgas (Messgas) geführt und anschließend detektiert wird. Die Wellenlänge des Lichts wird auf eine spezifische Absorptionslinie der jeweils zu messenden Gaskomponente abgestimmt, wobei die Laserdiode die Absorptionslinie periodisch wellenlängenabhängig abtastet. Dazu wird die Laserdiode innerhalb von periodisch aufeinanderfolgenden Abtastintervallen mit einem rampen- oder dreieckförmigen Stromsignal angesteuert. Während der vergleichsweise langsamen Abtastung der Absorptionslinie wird zusätzlich die Wellenlänge des erzeugten Lichts mit hoher Frequenz und kleiner Amplitude sinusförmig moduliert. Da das Profil der Absorptionslinie nicht linear ist, werden in dem bei der Detektion erhaltenen Messsignal auch Harmonische oberhalb der Modulationsfrequenz erzeugt. Das Messsignal wird üblicherweise bei einer n-ten Oberschwingung, vorzugsweise der zweiten Harmonischen, durch phasensensitive Lock-in Technik demoduliert und für jedes Abtastintervall zu einem Messergebnis ausgewertet. Bei kleiner Modulationsamplitude ist die Detektion der n-ten Harmonischen direkt proportional zu der n-ten Ableitung des direkten Messsignals. Die Auswertung erfolgt beispielsweise durch Anfitten (Curve-Fitting) des im Idealfall zu erwartenden Verlaufs des demodulierten Messsignals (Sollkurve) an dessen tatsächlichen Verlauf (Istkurve). Aus dem dabei erhaltenen Messergebnis wird schließlich die Konzentration der zu messenden Gaskomponente bestimmt.

Bei dem aus Cassidy D. T. et al bekannten Verfahren bzw. Gasanalysator wird die Wellenlänge des Lichts zusätzlich zu der Modulation mit der Frequenz f₁ mit einer Jitterfrequenz f₂ moduliert, um den Einfluss von Interferenzen auf das bei der zweiten Harmonischen demodulierte Messsignal zu verringern. Die Amplitude bzw. der Modulationsindex (d. h. das Verhältnis der spektralen Modulationsamplitude zur Halbwertsbreite der abgetasteten Absorptionslinie) der Modulation mit der Frequenz f₁ ist mit m₁ = 2,2 so gewählt, dass das demodulierte Messsignal an der Stelle der Absorptionslinie maximal ist. Die Jitterfrequenz f₂ und der Modulationsindex m₂ für die Modulation mit der Jitterfrequenz sind so gewählt, dass der Einfluss bestimmter Interferenzen minimal wird. Dabei ist die Jitterfrequenz grundsätzlich unabhängig von der Modulationsfrequenz und steht in keinem harmonischen Zusammenhang mit ihr. Lediglich für den Fall, dass die Breite der Absorptionslinie und die Abstände der Interferenzlinien vergleichbar sind, wird vorgeschlagen, die Jitterfrequenz f₂ = 3f₁ zu wählen; der Modulationsindex m₂ beträgt dabei m₂ = m₁/2. Die zweite Harmonische des demodulierten Messsignals setzt sich aus der zweiten Harmonischen 2f₁ des aus der Modulationsfrequenz f₁ resultierenden Messsignalanteils und den Summen- und Differenzfrequenzen f₂ - f₁, 2f₂ - 4f₁, 5f₁ - f₂, 3f₂ - 7f₁, 8f₁ - 24f₂ usw. zusammen.

Die Nachweis- und Bestimmungsgrenze für die Messung der Konzentration der Gaskomponente werden durch das Rauschen des Gasanalysators (z. B: Laserrauschen, Detektorrauschen) begrenzt, das sich dem Messsignal überlagert.

Der Erfindung liegt die Aufgabe zugrunde, das Messsignal-Rausch-Verhältnis zu verbessern.

Gemäß der Erfindung wird die Aufgabe durch das in Anspruch 1 definierte Verfahren sowie den in Anspruch 6 angegebenen Gasanalysator gelöst.

Vorteilhafte Weiterbildungen der Erfindung sind in den Unteransprüchen angegeben.

Entsprechend der Erfindung wird die Wellenlänge des Lichts der Lichtquelle nicht nur mit einer sondern mehreren Frequenzen (2n-1)f, n = 1, 2, 3..., moduliert. Das Messsignal wird bei den zweiten Harmonischen 2(2n-1)f dieser Frequenzen oder, wie unten noch erläutert wird, bei den Frequenzen 2nf, demoduliert. Die dabei erhaltenen demodulierten Messsignale werden beispielsweise durch Datenfusion (Data Fusion, Multi-Sensor Data Fusion) zusammengeführt, im einfachsten Fall addiert, und dann weiter, z. B. durch Kurvenauswertung, Curve-Fitting oder Korrelation mit Referenzsignalen, zu einem Messergebnis ausgewertet. Alternativ können zuerst die demodulierten Messsignale einzeln ausgewertet und anschließend die erhaltenen Einzel-Messergebnisse zu einem Gesamt-Messergebnis zusammengeführt, z. B. addiert werden.

Aufgrund der nichtlinearen Form der Absorptionslinie enthält das Messsignal nicht nur die Vielfachen (Harmonischen) der bei der Modulation verwendeten Frequenzen sondern auch die Summen- und Differenzen dieser Frequenzen. Da die Modulationsfrequenzen um den doppelten Betrag der kleinsten Modulationsfrequenz f auseinander liegen und die Demodulation des Messsignals bei den zweiten Harmonischen der Modulationsfrequenzen erfolgt, fallen die Summen- und Differenzfrequenzen entweder mit den zweiten Harmonischen der Modulationsfrequenzen zusammen oder liegen genau in der Mitte zwischen diesen, so dass die Frequenzanteile des Messsignals um den doppelten Betrag der kleinsten Modulationsfrequenz f und damit um die doppelte Signalbandbreite auseinander liegen. Die Amplituden der Frequenzen oberhalb der zweiten Harmonischen sind jeweils deutlich kleiner als die der zweiten Harmonischen und stören daher nicht. Damit ergeben sich bei Modulation der Wellenlänge des Lichts mit beispielsweise vier unterschiedlichen Frequenzen f, 3f, 5f, 7f folgende Frequenzanteile in dem Messsignal:
- 2f:: zweite Harmonische des aus der Modulationsfrequenz f resultierenden Messsignalanteils,
Differenz der Modulationsfrequenzen 3f und f,
Differenz der Modulationsfrequenzen 5f und 3f,
Differenz der Modulationsfrequenzen 7f und 5f;
- 4f:: Differenz der Modulationsfrequenzen 5f und f,
Differenz der Modulationsfrequenzen 7f und 3f,
Summe der Modulationsfrequenzen f und 3f;
- 6f:: zweite Harmonische des aus der Modulationsfrequenz 3f resultierenden Messsignalanteils,
Differenz der Modulationsfrequenzen 7f und f,
Summe der Modulationsfrequenzen f und 5f;
- 8f:: Summe der Modulationsfrequenzen f und 7f,
Summe der Modulationsfrequenzen 3f und 5f;
- 10f:: zweite Harmonische des aus der Modulationsfrequenz 5f resultierenden Messsignalanteils,
Summe der Modulationsfrequenzen 3f und 7f;
- 12f:: Summe der Modulationsfrequenzen 5f und 7f;
- 14f:: zweite Harmonische des aus der Modulationsfrequenz 7f resultierenden Messsignalanteils.

Da das Rauschen in den unterschiedlichen Frequenzbändern nicht korreliert ist und sich die unterschiedlichen Signalanteile bei den Frequenzen 2f bis 14f addieren, entsteht bei der Auswertung zu dem Messergebnis ein sehr hoher Signal-Rauschabstand.

Vorzugsweise werden alle Messsignalanteile mit den Frequenzen 2f bis 14f für die Auswertung genutzt. Es ist aber auch möglich nur die Messsignalanteile mit den aus den Modulationsfrequenzen f, 3f, 5f, 7f resultierenden zweiten Harmonischen 2f, 6f, 10f 14f zu nutzen. Auch kann im Falle von Störungen die Auswertung auf die jeweils besten Frequenzbänder begrenzt werden.

Wie bereits erwähnt, können die demodulierten Messsignale zunächst addiert und dann zu dem Messergebnis ausgewertet werden oder zuerst einzeln ausgewertet und danach die erhaltenen Einzel-Messergebnisse zu dem Gesamt-Messergebnis addiert werden. Ist das Rauschen auf beiden Varianten nicht korreliert, können beide Varianten berechnet und addiert werden, was zu einer weiteren Verbesserung des Signal-Rauschabstandes führen kann. Die Einzelauswertung der demodulierten Messsignale hat den Vorteil, dass die unterschiedlichen Frequenzmodulationen in Abhängigkeit von den einzelnen Messergebnissen korrigiert oder angepasst werden können. Im Übrigen hat jedoch eine Simulation gezeigt, dass die Summe der demodulierten Messsignale gut gefittet werden kann und das Ergebnis nicht wesentlich schlechter ist als das der addierten Einzel-Messergebnisse.

Zur weiteren Erläuterung der Erfindung wird im Folgenden auf die Figuren der Zeichnung Bezug genommen; im Einzelnen zeigen:
- Figur 1: ein erstes Ausführungsbeispiel des erfindungsgemäßen Gasanalysators,
- Figur 2: ein Beispiel für den Verlauf des Injektionsstroms einer Laserdiode in dem Gasanalysator, und
- Figur 3: ein zweites Ausführungsbeispiel des erfindungsgemäßen Gasanalysators.

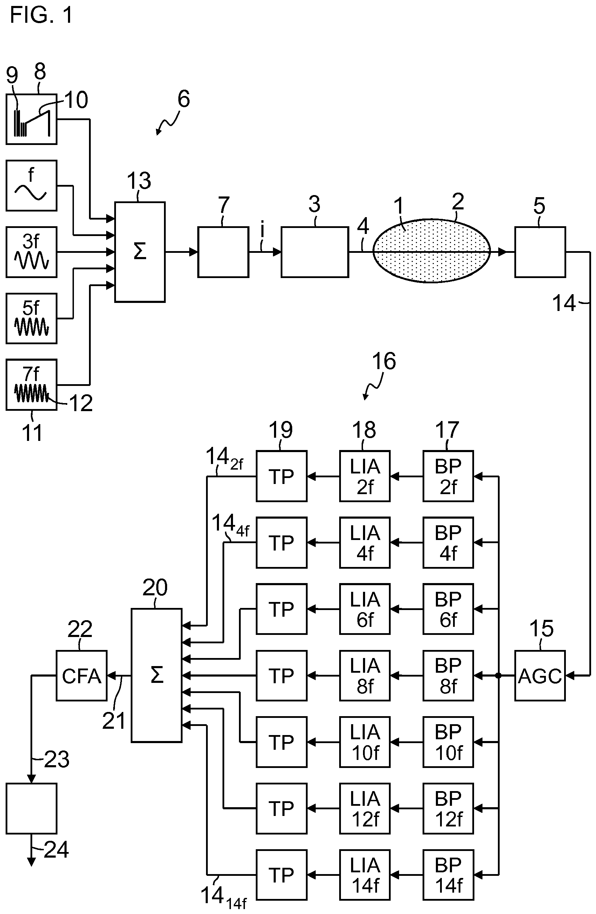

Bei dem in Figur 1 in Form eines vereinfachten Blockschaltbildes gezeigten Gasanalysator handelt es sich um ein Laserspektrometer zur Messung der Konzentration mindestens einer interessierenden Gaskomponente eines Messgases 1, das in einem Messvolumen 2, beispielsweise einer Messküvette oder einer Prozessgasleitung, enthalten ist. Das Spektrometer enthält eine Lichtquelle 3 in Form einer Laserdiode, deren Licht 4 nach Durchstrahlen des Messgases 1, auf einen Detektor 5 fällt. Eine von einer Modulationseinrichtung 6 gesteuerte Stromquelle 7 speist die Laserdiode 3 mit einem Injektionsstrom i, wobei die Intensität und Wellenlänge des erzeugten Lichts 4 von dem Strom i und der Betriebstemperatur der Laserdiode 3 abhängen. Die Modulationseinrichtung 6 umfasst einen ersten Signalgenerator 8, der die Stromquelle 7 periodisch mit einer vorgegebenen, vorzugsweise rampen- oder dreieckförmigen Funktion 10 angesteuert, um mit der mehr oder weniger linear dem Verlauf des Stromes i folgenden Wellenlänge des erzeugten Lichts 4 eine ausgewählte Absorptionslinie der interessierenden Gaskomponente abzutasten. Der erste Signalgenerator 8 erzeugt weiterhin regelmäßig, z. B. nach jeder Abtastperiode, ein Burstsignal 9. Mehrere, hier vier Signalgeneratoren 11 erzeugen sinusförmige Signale 12 höherer Frequenzen f, 3f, 5f, 7f, die in einem Summierglied 13 der rampen- oder dreieckförmigen Funktion 10 überlagert werden.

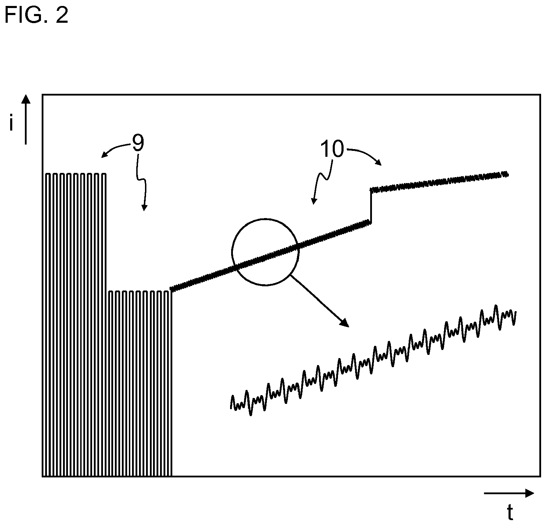

Figur 2 zeigt ein Beispiel für den Verlauf des Injektionsstroms i(t) während einer Ansteuerperiode. Mittels der rampenförmigen Funktion 10 werden hier zwei unterschiedliche Stromrampen erzeugt, um zwei unterschiedliche Absorptionslinien, z. B. die der zu messenden Gaskomponente des Messgases 1 und die eines Referenzgases (in Figur 1 nicht gezeigt), abtasten zu können. Die Stromrampen sind zusätzlich mit den Frequenzen f, 3f, 5f, 7f und kleiner Amplitude sinusförmig moduliert. Mit dem Burstsignal 9 werden zwei Strombursts erzeugt, deren Stärke dem Anfangs- bzw. Endwert der Stromrampe entspricht.

Zurück zu Figur 1 erzeugt der Detektor 5 in Abhängigkeit von der detektierten Lichtintensität ein Messsignal 14, das in einem Verstärker 15 hochpassgefiltert und anhand der aus dem Burstsignal 9 resultierenden Signalanteile automatisch verstärkt und normalisiert wird. In einer Auswerteeinrichtung 16 wird das normalisierte Messsignal 14 bei den zweiten Harmonischen 2f, 6f, 10f, 14f der Modulationsfrequenzen f, 3f, 5f, 7f und den dazwischen liegenden Summen- und Differenzfrequenzen 4f, 8f, 12f der Modulationsfrequenzen f, 3f, 5f, 7f demoduliert. Die Demodulation erfolgt in parallelen Kanälen, die jeweils ein Bandpassfilter 17 und einen Lock-in Verstärker 18 mit Tiefpassfilter 19 aufweisen. Dabei wird das bandpassgefilterte Messsignal 14 durch Multiplikation mit einem Referenzsignal bei der jeweiligen Demodulationsfrequenz 2f, 4f bis 14f phasensensitiv demoduliert und durch die anschließende Tiefpassfilterung die Inphasenkomponente, also der Nutzsignalanteil des demodulierten Messsignals 14_{2f}, 14_{4f} bis 14_{14f} extrahiert. Die demodulierten Messsignale 14_{2f}, 14_{4f} bis 14_{14f}, genauer ihre Nutzsignalanteile, werden in einem Addierer 20 zu einem Summensignal 21 aufsummiert und anschließend in einer Recheneinheit 22 ausgewertet. Die Auswertung erfolgt hier z. B. durch Anfitten (Curve-Fitting) des im Idealfall zu erwartenden Verlaufs des bei der zweiten Harmonischen demodulierten Messsignals an den Verlauf des Summensignals 21. (Nicht nur die zweiten Harmonischen der aus den unterschiedlichen Modulationsfrequenzen resultierenden Messsignalanteile sondern auch die aus den Differenzen und Summen der Modulationsfrequenzen resultierenden Messsignalanteile weisen jeweils den gleichen Verlauf auf.) Aus dem durch die Auswertung erhaltenen Gesamt-Messergebnis 23 wird schließlich die Konzentration 24 der zu messenden Gaskomponente bestimmt.

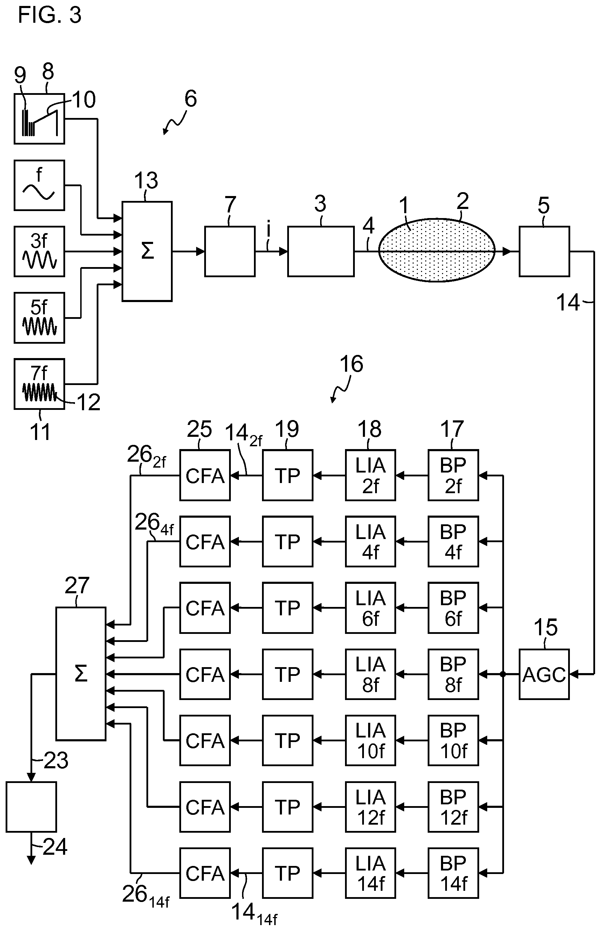

Figur 3 zeigt ein alternatives Ausführungsbeispiel des erfindungsgemäßen Gasanalysators, das sich von dem nach Figur 1 dadurch unterscheidet, dass die demodulierten Messsignale 14_{2f}, 14_{4f} bis 14_{14f} bzw. ihre Nutzsignalanteile in Recheneinheiten 25 einzeln gefittet werden und die Einzel-Messergebnisse 26_{2f}, 26_{4f} bis 26_{14f} des Curve-Fittings in einem Addierer 27 zu dem Gesamt-Messergebnis 23 aufsummiert werden.

Im Folgenden wird ein Zahlenbeispiel für die Auslegung des Gasanalysators gegeben:
Digitale Signalverarbeitung mit 192 kHz Abtastrate, 6 kHz Bandbreite, also Frequenzabstand 12 kHz und 4 Modulationsfrequenzen f = 6 kHz, 3f = 18 kHz, 5f = 30 kHz und 7f = 42 kHz. Für mehr Modulationsfrequenzen müsste die Signalverarbeitung analog erfolgen, die Abtastrate erhöht, oder die Bandbreite verkleinert werden. Auf der Detektorseite werden dann folgende Frequenzbänder benötigt: 2f = 12 kHz, 4f = 24 kHz, 6f = 36 kHz, 8f = 48 kHz, 10f = 60 kHz, 12f = 72 kHz und 14f = 84 kHz. Dies sind Vielfache der Abstände der Modulationsfrequenzen, hier also Vielfache von 2f = 12 kHz. Die Anzahl der Modulationsfrequenzen und damit die Anzahl der Frequenzbänder hängt letztlich von der verwendeten Lichtquelle ab, wobei zurzeit ein VCSEL-Laser mit bis zu einigen 100 kHz moduliert werden kann.

## Patentansprüche

1. Verfahren zur Messung der Konzentration einer Gaskomponente in einem Messgas (1) mittels eines Gasanalysators, wobei
- zur wellenlängenabhängigen Abtastung einer interessierenden Absorptionslinie der Gaskomponente die Wellenlänge des Lichts (4) einer wellenlängenabstimmbaren Lichtquelle (3) in periodisch aufeinanderfolgenden Abtastintervallen variiert und dabei zusätzlich mit einer ersten Modulationsfrequenz (f) und einer dreimal größeren zweiten Modulationsfrequenz (3f) sinusförmig moduliert wird,
- das modulierte Licht (4) durch das Messgas (1) auf einen Detektor (5) geführt wird, und
- ein von dem Detektor (5) erzeugtes Messsignal (14) bei der zweiten Harmonischen (2f) der Modulationsfrequenz (f) phasensensitiv demoduliert und ein dabei erhaltenes erstes demoduliertes Messsignal (14_{2f}) für jedes Abtastintervall zu einem ersten Einzel-Messergebnis (26_{2f}) ausgewertet wird,
**dadurch gekennzeichnet,**
- **dass** das Messsignal (14) zusätzlich bei der zweiten Harmonischen (6f) der zweiten Modulationsfrequenz (3f) phasensensitiv demoduliert wird und
- **dass** ein dabei erhaltenes zweites demoduliertes Messsignal (14_{6f}) entweder mit dem ersten demodulierten Messsignal (14_{2f}) zu einem Summensignal (21) zusammengeführt wird, das anschließend zu einem Gesamt-Messergebnis (23) ausgewertet wird, oder dass das zweite demodulierte Messsignal (14_{6f}) zu einem zweiten Einzel-Messergebnis (26_{6f}) ausgewertet wird, das mit dem ersten Einzel-Messergebnis (26_{2f}) zu dem Gesamt-Messergebnis (23) aufsummiert wird.

2. Verfahren nach Anspruch 1, **dadurch gekennzeichnet,**
- **dass** die Wellenlänge des Lichts (4) zusätzlich mit mindestens einer weiteren Modulationsfrequenz (5f, 7f) sinusförmig moduliert wird, die um den doppelten Betrag (2f) der ersten Modulationsfrequenz (f) größer als die nächst kleinere verwendete Modulationsfrequenz ist,
- **dass** das Messsignal (14) zusätzlich bei der zweiten Harmonischen (10f, 14f) der mindestens einen weiteren Modulationsfrequenz (5f, 7f) phasensensitiv demoduliert wird und
- **dass** ein dabei jeweils erhaltenes weiteres demoduliertes Messsignal (14_{10f}, 14_{14f}) entweder mit dem ersten und zweiten demodulierten Messsignal (14_{2f} 14_{6f}) zu dem Summensignal (21) zusammengeführt wird oder zu einem weiteren Einzel-Messergebnis (26_{10f}, 26_{14f}) ausgewertet wird, das mit dem ersten und zweiten Einzel-Messergebnis (26_{2f}, 26_{6f}) zu dem Gesamt-Messergebnis (23) aufsummiert wird.

3. Verfahren nach Anspruch 1 oder 2, **dadurch gekennzeichnet,**
- **dass** das Messsignal (14) zusätzlich bei mindestens einer der Summen- und Differenzfrequenzen (4f, 8f, 12f) der zur Modulation verwendeten Frequenzen (f, 3f, 5f, 7f) demoduliert wird und
- **dass** ein dabei erhaltenes zusätzliches demoduliertes Messsignal (14_{4f}, 14_{8f}, 14_{12f}) entweder mit dem ersten und zweiten demodulierten Messsignal (14_{2f}, 14_{6f}) und ggf. dem weiteren demodulierten Messsignal (14_{10f}, 14_{14f}) zu dem Summensignal (21) zusammengeführt wird oder zu einem zusätzlichen Einzel-Messergebnis (26_{4f}, 26_{8f}, 26_{12f}) ausgewertet wird, das mit dem ersten und zweiten Einzel-Messergebnis (26_{2f}, 26_{6f}) und ggf. dem weiteren Einzel-Messergebnis (26_{10f}, 26_{14f}) zu dem Gesamt-Messergebnis (23) aufsummiert wird.

4. Verfahren nach einem der vorangehenden Ansprüche, **dadurch gekennzeichnet, dass** zur Auswertung des Summensignals (21), des ersten und zweiten demodulierten Messsignals (14_{2f}, 14_{6f}), des weiteren demodulierten Messsignals (14_{10f}, 14_{14f}) oder des zusätzlichen demodulierten Messsignals (14_{4f}, 14_{8f}, 14_{12f}) an deren Verläufe über das Abtastintervall jeweils eine Idealkurve angefittet wird

5. Verfahren nach einem der vorangehenden Ansprüche,
**dadurch gekennzeichnet, dass** die Zusammenführung durch Datenfusion oder Addition erfolgt.

6. Gasanalysator zur Messung der Konzentration einer Gaskomponente in einem Messgas (1) mit
- einer wellenlängenabstimmbaren Lichtquelle (3),
- einer Modulationseinrichtung (6), die zur wellenlängenabhängigen Abtastung einer interessierenden Absorptionslinie der Gaskomponente die Wellenlänge des Lichts (4) der Lichtquelle (3) innerhalb periodisch aufeinanderfolgender Abtastintervalle variiert und dabei zusätzlich mit einer Modulationsfrequenz (f) und einer dreimal größeren zweiten Modulationsfrequenz (3f) moduliert,
- einem Detektor,
- Mitteln, die das modulierte Licht (4) durch das Messgas (1) auf den Detektor (5) führen, und
- einer Auswerteeinrichtung (16), die ein von dem Detektor (5) erzeugtes Messsignal (14) bei der zweiten Harmonischen (2f) der Frequenz (f) phasensensitiv demoduliert und ein dabei erhaltenes erstes demoduliertes Messsignal (14_{2f}) für jedes Abtastintervall zu einem ersten Einzel-Messergebnis (26_{2f}) auswertet,
**dadurch gekennzeichnet,**
- **dass** die Auswerteeinrichtung (16) dazu ausgebildet ist, das Messsignal (14) zusätzlich bei der zweiten Harmonischen (6f) der zweiten Modulationsfrequenz (3f) phasensensitiv zu demodulieren und
- ein dabei erhaltenes zweites demoduliertes Messsignal (14_{6f}) entweder mit dem ersten demodulierten Messsignal (14_{2f}) zu einem Summensignal (21) zusammenzuführen und dieses zu einem Gesamt-Messergebnis (23) auszuwerten, oder das zweite demodulierte Messsignal (14_{6f}) zu einem zweiten Einzel-Messergebnis (26_{6f}) auszuwerten und dieses mit dem ersten Einzel-Messergebnis (26_{2f}) aufzusummieren.

7. Gasanalysator nach Anspruch 6, **dadurch gekennzeichnet, dass** die Modulationseinrichtung (6) ferner dazu ausgebildet ist, die Wellenlänge des Lichts (4) zusätzlich mit mindestens einer weiteren Modulationsfrequenz (5f, 7f) sinusförmig zu modulieren, die um den doppelten Betrag (2f) der ersten Modulationsfrequenz (f) größer als die nächst kleinere verwendete Modulationsfrequenz ist, und
dass die Auswerteeinrichtung (16) ferner dazu ausgebildet ist, das Messsignal (14) zusätzlich bei der zweiten Harmonischen (10f, 14f) der mindestens einen weiteren Modulationsfrequenz (5f, 7f) phasensensitiv zu demodulieren und ein dabei jeweils erhaltenes weiteres demoduliertes Messsignal (14_{10f}, 14_{14f}) entweder mit dem ersten und zweiten demodulierten Messsignal (14_{2f} 14_{6f}) zu dem Summensignal (21) zusammenzuführen oder zu einem weiteren Einzel-Messergebnis (26_{10f}, 26_{14f}) auszuwerten und dieses mit dem ersten und zweiten Einzel-Messergebnis (26_{2f} 26_{6f}) zu dem Gesamt-Messergebnis (23) aufzusummieren.

8. Gasanalysator nach Anspruch 6 oder 7, **dadurch gekennzeichnet,**
**dass** die Auswerteeinrichtung (16) ferner dazu ausgebildet ist, das Messsignal (14) zusätzlich bei mindestens einer der Summen- und Differenzfrequenzen (4f, 8f, 12f) der Modulationsfrequenzen (f, 3f, 5f, 7f) zu demodulieren und ein dabei erhaltenes zusätzliches demoduliertes Messsignal (14_{4f}, 14_{8f}, 14_{12f}) entweder mit dem ersten und zweiten demodulierten Messsignal (14_{2f}, 14_{6f}) und ggf. dem weiteren demodulierten Messsignal (14_{10f}, 14_{14f}) zu dem Summensignal (21) zusammenzuführen oder zu einem zusätzlichen Messergebnis (26_{4f}, 26_{8f}, 26_{12f}) auszuwerten und dieses mit dem ersten und zweiten Einzel-Messergebnis (26_{2f}, 26_{6f}) und ggf. dem weiteren Einzel-Messergebnis (26_{10f}, 26_{14f}) zu dem Gesamt-Messergebnis (23)
aufzusummieren.

## Claims

1. Method for measuring the concentration of a gas component in a sample gas (1) by means of a gas analyzer, wherein,
- for the purposes of wavelength-dependent sampling of an absorption line of interest of the gas component, the wavelength of the light (4) of a wavelength-tunable light source (3) is varied in periodically successive sampling intervals and, in the process, there additionally is sinusoidal modulation with a first modulation frequency (f) and a second modulation frequency (3f) which is three times greater,
- the modulated light (4) is guided through the sample gas (1) to a detector (5), and
- a measurement signal (14) generated by the detector (5) is demodulated in a phase-sensitive manner at the second harmonic (2f) of the modulation frequency (f) and a first demodulated measurement signal (14_{2f}) obtained in the process is evaluated to form a first individual measurement result (26_{2f}) for each sampling interval,
**characterized**
- **in that** the measurement signal (14) is additionally demodulated in a phase-sensitive manner at the second harmonic (6f) of the second modulation frequency (3f) and
- **in that** a second demodulated measurement signal (14_{6f}) obtained in the process is either combined with the first demodulated measurement signal (14_{2f}) to form a sum signal (21), which is then evaluated to form an overall measurement result (23), or in that the second demodulated measurement signal (14_{6f}) is evaluated to form a second individual measurement result (26_{6f}) which is summed with the first individual measurement result (26_{2f}) to form the overall measurement result (23).

2. Method according to Claim 1, **characterized**
- **in that** the wavelength of the light (4) is additionally modulated in a sinusoidal manner with at least one further modulation frequency (5f, 7f) which, by twice the magnitude (2f) of the first modulation frequency (f), is greater than the next smallest modulation frequency used,
- **in that** the measurement signal (14) is additionally demodulated in a phase-sensitive manner at the second harmonic (10f, 14f) of the at least one further modulation frequency (5f, 7f) and
- **in that** a further demodulated measurement signal (14_{10f}, 14_{14f}) obtained in each case in the process is either combined with the first and second demodulated measurement signal (14_{2f}, 14_{6f}) to form the sum signal (21) or evaluated to form a further individual measurement result (26_{10f}, 26_{14f}) which is summed with the first and second individual measurement result (26_{2f}, 26_{6f}) to form the overall measurement result (23).

3. Method according to Claim 1 or 2, **characterized**
- **in that** the measurement signal (14) is additionally demodulated at at least one of the sum and difference frequencies (4f, 8f, 12f) of the frequencies (f, 3f, 5f, 7f) used for the modulation and
- **in that** an additional demodulated measurement signal (14_{4f}, 14_{8f}, 14_{12f}) obtained in the process is either combined with the first and second demodulated measurement signal (14_{2f}, 14_{6f}) and optionally the further demodulated measurement signal (14_{10f}, 14_{14f}) to form the sum signal (21) or evaluated to form an additional individual measurement result (26_{4f}, 26_{8f}, 26_{12f}) which is summed with the first and second individual measurement result (26_{2f}, 26_{6f}) and optionally the further individual measurement result (26_{10f}, 26_{14f}) to form the overall measurement result (23).

4. Method according to one of the preceding claims, **characterized in that**, for the purposes of evaluating the sum signal (21), the first and second demodulated measurement signal (14_{2f}, 14_{6f}), the further demodulated measurement signal (14_{10f}, 14_{14f}) or the additional demodulated measurement signal (14_{4f}, 14_{8f}, 14_{12f}), in each case an ideal curve is fitted to the profiles thereof over the sampling interval.

5. Method according to one of the preceding claims, **characterized in that** combining is brought about by data fusion or addition.

6. Gas analyzer for measuring the concentration of a gas component in a sample gas (1), comprising
- a wavelength-tunable light source (3),
- a modulation apparatus (6) which, for the purposes of wavelength-dependent sampling of an absorption line of interest of the gas component, varies the wavelength of the light (4) of the light source (3) within periodically successive sampling intervals and, in the process, additionally modulates this with a modulation frequency (f) and a second modulation frequency (3f) which is three times greater,
- a detector,
- means which guide the modulated light (4) through the sample gas (1) to the detector (5), and
- an evaluation apparatus (16) which demodulates a measurement signal (14) generated by the detector (5) at the second harmonic (2f) of the frequency (f) in a phase-sensitive manner and evaluates a first demodulated measurement signal (14_{2f}) obtained in the process to form a first individual measurement result (26_{2f}) for each sampling interval,
**characterized**
- **in that** the evaluation apparatus (16) is embodied to additionally demodulate the measurement signal (14) at the second harmonic (6f) of the second modulation frequency (3f) in a phase-sensitive manner and
- either to combine a second demodulated measurement signal (14_{6f}) obtained in the process with the first demodulated measurement signal (14_{2f}) to form a sum signal (21) and to evaluate the latter to form an overall measurement result (23), or to evaluate the second demodulated measurement signal (14_{6f}) to form a second individual measurement result (26_{6f}) and to sum the latter with the first individual measurement result (26_{2f}).

7. Gas analyzer according to Claim 6, **characterized**
**in that** the modulation apparatus (6) is further embodied to additionally modulate the wavelength of the light (4) in a sinusoidal manner with at least one further modulation frequency (5f, 7f) which, by twice the magnitude (2f) of the first modulation frequency (f), is greater than the next smallest modulation frequency used, and
**in that** the evaluation apparatus (16) is further embodied to additionally demodulate the measurement signal (14) in a phase-sensitive manner at the second harmonic (10f, 14f) of the at least one further modulation frequency (5f, 7f) and either to combine a further demodulated measurement signal (14_{10f}, 14_{14f}) obtained in each case in the process with the first and second demodulated measurement signal (14_{2f}, 14_{6f}) to form the sum signal (21) or to evaluate it to form a further individual measurement result (26_{10f}, 26_{14f}) and to sum the latter with the first and second individual measurement result (26_{2f}, 26_{6f}) to form the overall measurement result (23).

8. Gas analyzer according to Claim 6 or 7, **characterized**
**in that** the evaluation apparatus (16) is further embodied to additionally demodulate the measurement signal (14) at at least one of the sum and difference frequencies (4f, 8f, 12f) of the modulation frequencies (f, 3f, 5f, 7f) and either to combine an additional demodulated measurement signal (14_{4f}, 14_{8f}, 14_{12f}) obtained in the process with the first and second demodulated measurement signal (14_{2f}, 14_{6f}) and optionally the further demodulated measurement signal (14_{10f}, 14_{14f}) to form the sum signal (21) or to evaluate it to form an additional measurement result (26_{4f}, 26_{8f}, 26_{12f}) and to sum the latter with the first and second individual measurement result (26_{2f}, 26_{6f}) and optionally the further individual measurement result (26_{10f}, 26_{14f}) to form the overall measurement result (23).

## Revendications

1. Procédé de mesure de la concentration d'un constituant gazeux dans un gaz (1) à mesurer au moyen d'un analyseur de gaz, dans lequel
- pour l'échantillonnage en fonction de la longueur d'onde d'une raie d'absorption intéressante du constituant gazeux, on fait varier la longueur d'onde de la lumière (4) d'une source (3) lumineuse accordable en longueur d'onde à des intervalles d'échantillonnage se succédant périodiquement et on module ainsi sinusoïdalement supplémentairement à une première fréquence (f) de modulation et à une deuxième fréquence (3f) de modulation trois fois plus grande,
- on fait passer la lumière (4) modulée dans le gaz (1) à mesurer jusqu'à un détecteur (5) et
- on démodule, d'une manière sensible à la phase, un signal (14) de mesure produit par le détecteur (5) au deuxième harmonique (2f) de la fréquence (f) de modulation et on exploite un premier signal (14_{2f}) de mesure démodulé ainsi obtenu pour chaque intervalle d'échantillonnage, en un premier résultat (26_{2f}) de mesure individuel,
**caractérisé**
- **en ce que** l'on démodule d'une manière sensible à une phase, le signal (14) de mesure supplémentairement au deuxième harmonique (6f) de la deuxième fréquence (3f) de modulation et
- **en ce que** l'on rassemble, en un signal (21) de somme, un deuxième signal (14_{6f}) de mesure démodulé ainsi obtenu, soit avec le premier signal (14_{2f}) de mesure démodulé, que l'on exploite ensuite en un résultat (23) de mesure d'ensemble, soit que l'on exploite le deuxième signal (14_{6f}) de mesure démodulé en un deuxième résultat (26_{6g}) de mesure individuel, que l'on somme avec le premier résultat (26_{2f}) de mesure individuel en le résultat (23) de mesure d'ensemble.

2. Procédé suivant la revendication 1, **caractérisé**
- **en ce que** l'on module sinusoïdalement la longueur d'onde de la lumière (4) supplémentairement à au moins une autre fréquence (5f, 7f) de modulation, qui est plus grande du double de la valeur (2f) de la première fréquence (f) de modulation que la fréquence de modulation utilisée immédiatement plus petite,
- **en ce que** l'on démodule, d'une manière sensible à une phase, le signal (14) de mesure supplémentairement au deuxième harmonique (10f, 14f) de la au moins une autre fréquence (5f, 7f) de modulation et
- **en ce que** l'on rassemble un autre signal (14_{10f}, 14_{14f}) de mesure démodulé ainsi obtenu, soit avec le premier et le deuxième signal (14_{2f}, 14_{6f}) de mesure démodulé en le signal (21) somme, soit on l'exploite en un autre résultat (26_{10f}, 26_{14f}) de mesure individuel que l'on somme avec le premier et le deuxième résultat (26_{2f}, 26_{6f}) de mesure individuel en le résultat (23) de mesure d'ensemble.

3. Procédé suivant la revendication 1 ou 2, **caractérisé**
- **en ce que** l'on démodule le signal (14) de mesure supplémentairement à au moins l'une des fréquences (4f, 8f, 12f) de somme et de différence des fréquences (f, 3f, 5f, 7f) utilisées pour la modulation et
- **en ce que** l'on rassemble un signal (14_{4f}, 14_{8f}, 14_{12f}) de mesure démodulé supplémentaire ainsi obtenu, soit avec le premier et le deuxième signal (14_{2f}, 14_{6f}) de mesure démodulé et, le cas échéant, l'autre signal (14,_{10f}, 14_{14f}) de mesure démodulé en le signal (21) somme, ou on l'exploite en un résultat (26_{4f}, 26_{8f}, 26_{12f}) de mesure individuel supplémentaire, que l'on somme avec le premier et le deuxième résultat (26_{2f}, 26_{6f}) de mesure individuel et, le cas échéant, l'autre résultat (26_{10f}, 26_{14f}) de mesure individuel en le résultat (23) de mesure d'ensemble.

4. Procédé suivant l'une des revendications précédentes, **caractérisé en ce que**, pour l'exploitation du signal (21) de somme du premier et du deuxième signal (14_{2f}, 14_{6f}) de mesure démodulé de l'autre signal (14_{10f}, 14_{14f}) de mesure démodulé ou du signal (14_{4f}, 14_{8f}, 14_{12f}) de mesure démodulé supplémentaire, on ajuste sur leur courbe, sur l'intervalle d'échantillonnage, respectivement une courbe idéale.

5. Procédé suivant l'une des revendications précédentes, **caractérisé en ce que** l'on effectue le rassemblement par fusion de données ou pour addition.

6. Analyseur de gaz pour mesurer la concentration d'un constituant gazeux d'un gaz (1) à mesurer, comprenant
- une source (3) lumineuse accordable en longueur d'onde,
- un dispositif (6) de modulation, qui, pour l'échantillonnage en fonction de la longueur d'onde d'une raie d'absorption intéressante du constituant gazeux, fait varier la longueur d'onde de la lumière (4) de la source (3) lumineuse dans un intervalle d'échantillonnage se reproduisant périodiquement et module supplémentairement à une fréquence (f) de modulation et à une deuxième fréquence (3f) de modulation trois fois plus grande,
- un détecteur,
- des moyens, qui font passer la lumière (4) modulée dans le gaz (1) à mesurer jusqu'au détecteur (5) et
- un dispositif (16) d'exploitation, qui démodule, d'une manière sensible à une phase, un signal (14) de mesure produit par le détecteur (5) au deuxième harmonique (2f) de la fréquence (f) et exploite un premier signal (14_{2f}) de mesure démodulé ainsi obtenu pour chaque intervalle d'échantillonnage, en un premier résultat (26_{2f}) de mesure individuel,
**caractérisé**
- **en ce que** le dispositif (16) d'exploitation est constitué pour démoduler, d'une manière sensible à une phase, le signal (14) de mesure supplémentairement au deuxième harmonique (6f) de la deuxième fréquence (3f) de modulation et
- pour rassembler un deuxième signal (14_{6f}) de mesure démodulé ainsi obtenu, soit avec le premier signal (14_{2f}) de mesure démodulé en un signal (21) de somme et exploiter celui-ci en un résultat (23) de mesure d'ensemble, soit exploiter le deuxième signal (14_{6f}) de mesure démodulé en un deuxième résultat (26_{6f}) de mesure individuel et sommer celui-ci avec le premier résultat (26_{2f}) de mesure individuel.

7. Analyseur de gaz suivant la revendication 6, **caractérisé en ce que** le dispositif (6) à modulation est constitué, en outre, pour moduler sinusoïdalement la longueur d'onde de la lumière (4) supplémentairement à au moins une autre fréquence (5f, 7f) de modulation, qui, par la valeur (2f) double de la première fréquence (f) de modulation, est plus grande que la fréquence de modulation utilisée la plus petite ensuite et
**en ce que** le dispositif (16) d'exploitation est constitué, en outre, pour démoduler, d'une manière sensible à une phase, le signal (14) de mesure supplémentairement aux deuxièmes harmoniques (10f, 14f) de la au moins une autre fréquence (5f, 7f) de modulation et pour rassembler un autre signal (14_{10f}, 14_{14f}) de mesure démodulé ainsi obtenu, soit avec le premier et le deuxième signal (14_{2f}, 14_{6f}) de mesure démodulé en le signal (21) de somme, soit exploiter en un autre résultat (26_{10f}, 26_{14f}) de mesure individuel et sommer celui-ci avec le premier et le deuxième résultat (26_{2f}, 26_{6f}) de mesure individuel en le résultat (23) de mesure d'ensemble.

8. Analyseur de gaz suivant la revendication 6 ou 7, **caractérisé**
**en ce que** le dispositif (16) d'exploitation est constitué, en outre, pour démoduler le signal (14) de mesure supplémentairement à au moins l'une des fréquences (4f, 8f, 12f) de somme et de différence de fréquences (f, 3f, 5f, 7f) de modulation et à rassembler un signal (14_{4f}, 14_{8f}, 14_{12f}) de mesure démodulé supplémentaire ainsi obtenu, soit avec le premier et le deuxième signal (14_{2f}, 14_{6f}) de mesure démodulé et, le cas échéant, l'autre signal (14_{10f}, 14_{14f}) de mesure démodulé en le signal (21) de somme, soit l'exploiter en un résultat (26_{4f}, 26_{8f}, 26_{12f}) de mesure supplémentaire et sommer celui-ci avec le premier et le deuxième résultat (26_{2f}, 26_{6f}) de mesure individuel et, le cas échéant, l'autre résultat (26_{10f}, 26_{14f}) de mesure supplémentaire en le résultat (23) de mesure d'ensemble.
